# EUROPEAN PATENT SPECIFICATION

(11) **EP 1 311 259 B1**
(45) Date of publication and mention of the grant of the patent: **15.06.2005**
(21) Application number: 01984399.4
(22) Date of filing: 25.07.2001
(51) Int. Cl.: A61K 31/195, A61K 47/32, A61K 47/02, A61K 47/36, A61K 47/38, A61P 43/00

(54) **PHARMACEUTICAL COMPOSITION COMPRISING AN ESTER OF 5-AMINOLEVULINIC ACID AS PHOTOCHEMOTHERAPEUTIC AGENT AND A MUCOADHESIVE AGENT**
PHARMAZEUTISCHE ZUSAMMENSETZUNG ENTHALTEND EIN 5-AMINOLAEVULINSÄURE-ESTER ALS PHOTOCHEMOTHERAPEUTIKUM UND EIN MUCOADHÄSIVUM
COMPOSITION PHARMACEUTIQUE COMPRENANT UN ESTER DE L'ACIDE 5-AMINOLEVULINIC COMME AGENT PHOTOSENSIBILISATEUR ET UN AGENT MUCOADHESIF

(30) Priority: 27.07.2000 GB 0018527
(43) Date of publication of application: 21.05.2003
(73) Proprietor: Photocure ASA, 0377 Oslo (NO)
(72) Inventor: KLAVENESS, Jo, N-1166 Oslo (NO); HANSSON, Vidar, N-1336 Blommenholm (NO); GODAL, Aslak, N-0372 Oslo (NO)
(74) Representative: Jones, Elizabeth Louise
(86) International application number: PCT/GB2001/003338
(87) International publication number: WO 2002/009690

(56) References cited:
- EP-A- 0 911 023
- WO-A-95/05813
- WO-A-98/30242
- CH-A- 686 166
- US-A- 5 576 013

## Description

The present invention relates to pharmaceutical compositions for use in the treatment or diagnosis of disorders or abnormalities of epithelial-lined body surfaces, particularly mucosa-lined surfaces, by photochemotherapy with ALA esters or derivatives thereof in combination with mucoadhesive agents.

Photochemotherapy, or photodynamic therapy (PDT) as it is also known, is a technique for the treatment of various abnormalities or disorders of the skin or other epithelial organs, especially cancers or pre-cancerous lesions, as well as certain non-malignant lesions for example skin complaints such as psoriasis. Photochemotherapy involves the application of photosensitizing (photochemotherapeutic) agents to the affected area of the body, followed by exposure to photoactivating light in order to activate the photosensitizing agents and convert them into cytotoxic form, whereby the affected cells are killed or their proliferative potential diminished.

A range of photosensitizing agents are known, including notably the psoralens, the porphyrins, the chlorins and the phthalocyanins. Such drugs become toxic when exposed to light.

Photosensitizing drugs may exert their effects by a variety of mechanisms, directly or indirectly. Thus for example, certain photosensitizers become directly toxic when activated by light, whereas others act to generate toxic species, e.g. oxidising agents such as singlet oxygen or other oxygen-derived free radicals, which are extremely destructive to cellular material and biomolecules such as lipids, proteins and nucleic acids. Psoralens are an example of directly acting photosensitizers; upon exposure to light they form adducts and cross-links between the two strands of DNA molecules, thereby inhibiting DNA synthesis. The unfortunate risk with this therapy is that unwanted mutagenic and carcinogenic side effects may occur.'

This disadvantage may be avoided by selecting photosensitizers with an alternative, indirect mode of action. For example porphyrins, which act indirectly by generation of toxic oxygen species, have no mutagenic side effects and represent more favourable candidates for photochemotherapy. Porphyrins are naturally occurring precursors in the synthesis of heme. In particular, heme is produced when iron (Fe³⁺) is incorporated in protoporphyrin IX (Pp) by the action of the enzyme ferrochelatase. Pp is an extremely potent photosensitizer, whereas heme has no photosensitizing effect.

One such porphyrin-based drug, Photofrin®, has recently been approved as a photosensitizer in the therapy of certain cancers. The main disadvantage is that since it must be administered parenterally, generally intravenously, it may cause photosensitization of the skin which may last for several weeks following i.v. injection. Photofrin® consists of large oligomers of porphyrin and it does not readily penetrate the skin when applied topically. Similar problems exist with other porphyrin-based photosensitizers such as the so-called "hematoporphyrin derivative" (Hpd) which has also been reported for use in cancer photochemotherapy (see for example S. Dougherty. J. Natl. Cancer Ins., 1974, 52; 1333; Kelly and Snell, J. Urol, 1976, 115 : 150). Hpd is a complex mixture obtained by treating haematoporphyrin with acetic and sulphuric acids, after which the acetylated product is dissolved with alkali. Clearly, there are disadvantages in using an undefined mixture as a drug. Moreover since Hpd must also be administered by injection, it suffers from the same type of undesirable photosensitization drawback as does Photofrin®.

To overcome these problems, precursors of Pp have been investigated for photochemotherapeutic potential. In particular the Pp precursor 5-aminolevulinic acid (ALA) has been investigated as a photochemotherapeutic agent for certain skin cancers. ALA, which is formed from succinyl CoA and glycine in the first step of heme synthesis, is to a limited extent able to penetrate the skin and lead to a localised build-up of Pp; since, the action of ferrochelatase (the metallating enzyme) is the rate limiting step in heme synthesis, an excess of ALA leads to accumulation of Pp, the photosensitizing agent. Thus, by applying ALA topically to skin tumours, and then after several hours exposing the tumours to light, a beneficial photochemotherapeutic effect may be obtained (see for example WO91/01727). Since the skin covering basilomas and squamous cell carcinomas is more readily penetrated by ALA than healthy skin, and since the concentration of ferrochelatase is low in skin tumours, it has been found that topical application of ALA leads to a selectively enhanced production of Pp in tumours.

However, photochemotherapy with ALA is not always entirely satisfactory. ALA is not able to penetrate all tumours and other tissues with sufficient efficacy to enable treatment of a wide range of tumours or other conditions and ALA also tends to be unstable in pharmaceutical formulations. These problems have to some extent been overcome by the use of ALA esters which exhibit advantageous properties such as improved selectivity for abnormal tissue, non-systemic localization of administered agents, improved uptake and PpIX production and reduced pain sensation on administration (see WO98/30242 and WO96/28412). Database Xfire, entries 3060978, 5347132, 5499790, 5620924, 5633390, 5991317 and 6517740 (Beilstein); Cosmo Sogo Kenkyusho KK, Patent Abstracts of Japan, Vol 16; No. 156 (C-0930), 16.4.1992; EP-A-316179 (Tokuyama Soda KK); GB-A-2058077 (Hudson et al) and DE-A-2411382 (Boehringer Sohn Ingelheim) describe alkyl ester derivatives of 5-aminolevulinic acid, and derivatives and salts thereof and processes for their preparation.

However, these compounds still exhibit some limitations for treating all abnormalities or disorders and there is thus still a need for better photochemotherapeutic agents to treat abnormalities or disorders.

The present invention addresses this need and in particular aims to provide photochemotherapeutic agents in compositions which exhibit improved properties for the treatment of epithelial-lined body surfaces, particularly mucosa-lined surfaces.

It has now surprisingly been found that the use of bioadhesive agents, particularly mucoadhesive agents, in combination with ALA esters or derivatives thereof as photochemotherapeutic agents offers significant advantages in treating or diagnosing abnormalities, disorders or conditions on epithelial-lined body surfaces, particularly mucosa-lined surfaces.

Bioadhesives are a class of molecules which are known to form an interaction with materials of a biological nature for an extended period of time by interfacial forces resulting in fixation to a specific biological location. Attachment is achieved by complex but non-specific mechanisms, generally via non-covalent binding such as electrostatic forces, van der Waals forces, hydrogen bonds and hydrophobic interactions. (For reviews on the subject see for example Lehr, 1996, Eur. J. Drug Metab. Pharmacokin., 21(2), p139-148; Lehr, 1994, Crit. Rev. Therap. Drug Carrier Syst., 11(2&3), p119-160.)

Mucoadhesive agents are a special class of bioadhesive agents which bind to mucous, particularly the glycoprotein mucin which is present in the mucous layer. Mucous is produced in the eye, ear, nose and mouth. It also lines the respiratory, gastrointestinal and reproductive tracts primarily for protection and lubrication. The mucous membrane (or mucosa) may be of varying thickness and may be smooth or rough and may be covered with villosities, microvillosities or cilia.

Generally the attachment of the mucoadhesive to mucin is sufficiently strong so that removal occurs primarily through mucin turnover, ie. the weaker bond is the mucin-mucin rather than the mucin-mucoadhesive bond. In addition, some agents which exhibit an affinity for mucous surfaces bind to the epithelial cells which lie beneath the mucous layer, e.g. lectins, by virtue of specific receptor-mediated interactions. As used herein, "mucoadhesive agents" refers to agents which exhibit an affinity for a mucosa surface, ie. adhere to that surface through the formation of bonds (generally non-covalent in nature), whether binding occurs through interaction with the mucous and/or the underlying cells.

In a first aspect, the present invention provides a composition, preferably a pharmaceutical composition, comprising an ALA ester or a derivative thereof as a photochemotherapeutic agent and a mucoadhesive agent, as described herein, optionally together with at least one surface penetration assisting agent and optionally with one or more chelating agents, optionally together with at least one pharmaceutically acceptable carriers or excipients. It will be appreciated from the discussion herein that the mucoadhesive agent may itself comprise the carrier or excipient and thus in those cases, a further carrier or excipient is optionally present.

In a further aspect, the present invention provides a pharmaceutical composition for use as a medicament, preferably for the treatment or diagnosis of disorders or abnormalities of epithelial-lined surfaces, preferably mucosa-lined surfaces, comprising an ALA ester or a derivative thereof as a photochemotherapeutic agent together with a mucoadhesive agent, optionally together with at least one surface penetration assisting agent and optionally with one or more chelating agents. This achieves enhanced photochemotherapy when compared to the use of the photochemotherapeutic agent alone.

Alternatively viewed, the invention can be seen to provide the use of an ALA ester or a derivative thereof as a photochemotherapeutic agent together with a mucoadhesive, optionally together with at least one surface penetration assisting agent and optionally with one or more chelating agents in the preparation of a composition for the treatment or diagnosis of disorders or abnormalities of epithelial-lined surfaces, preferably mucosa-lined surfaces.

As mentioned above, the invention extends to novel compositions of ALA esters or derivatives thereof as photochemotherapeutic agents and mucoadhesives, optionally together with at least one surface penetration assisting agent and optionally with one or more chelating agents. These different components of the composition may be administered by different routes and/or at different times. Thus, viewed from a further aspect, the invention provides a product comprising an ALA ester or a derivative thereof as a photochemotherapeutic agent and a mucoadhesive, optionally together with at least one surfacle penetration assisting agent and optionally one or more chelating agents as a combined preparation for simultaneous, separate or sequential use in treating or diagnosing disorders or abnormalities of epithelial-lined surfaces, preferably mucosa-lined surfaces.

Furthermore, the use of an ALA ester or a derivative thereof as a photochemotherapeutic agent and a mucoadhesive, optionally together with at least one surface penetration assisting agent and optionally one or more chelating agents in the preparation of a product for simultaneous, separate or sequential use in treating or diagnosing disorders or abnormalities of epithelial-lined surfaces, preferably mucosa-lined surfaces, forms a further aspect of the invention.

As used herein, "photochemotherapeutic agent" refers to ALA esters or derivatives thereof which are photosensitive, ie. photosensitizers and which on the application of photoactivating light are converted to a cytotoxic form or give rise to a cytotoxic species.

"Derivatives" include pharmaceutically acceptable salts and chemically modified agents. Preferably, ALA esters and their derivatives comprise a compound of general formula I,

R² ₂N-CH₂COCH₂-CH₂CO-OR¹ (I)

(wherein
R¹ and R² each independently represents a hydrogen atom or an optionally substituted straight-chained, branched or cyclic alkyl group which may optionally be interrupted by one or more -O-, -NR³-, -S- or -PR³- groups, with the proviso that R¹ is other than hydrogen; and
R³ is a hydrogen atom or a C₁₋₆ alkyl group) and pharmaceutically acceptable salts thereof.

As used herein, unless specifically stated otherwise, the term "alkyl" includes any long or short chain, straight-chained, branched or cyclic aliphatic saturated or unsaturated hydrocarbon group optionally mono or poly substituted by hydroxy, alkoxy, acyloxy, nitro, alkoxycarbonyloxy, amino, aryl, oxo, halo (e.g. fluoro or chloro) groups, -SR³, -NR³₂, or PR³₂ groups (in which R³ is as hereinbefore defined). The unsaturated alkyl groups may be mono- or polyunsaturated and include both alkenyl and alkynyl groups. Such groups may contain up to 40, but preferably 30, e.g. 7 to 30 carbon atoms. However, alkyl groups containing up to 20, eg. up to 10, e.g. from 3 to 10 carbon atoms, more preferably from 6 to 8 carbon atoms are preferred. In particular, straight-chained saturated hydrocarbons having up to 10 carbon atoms are preferred, e.g. hexyl, heptyl or octyl groups. Lower alkyls such as methyl, ethyl and propyl may, however, alternatively be used.

As used herein heterocyclic rings are preferably C₆₋₇ and optionally contain one or more further heteroatoms selected from oxygen, nitrogen and sulphur.

The substituted alkyl groups may be mono or polysubstituted. Thus suitable groups R¹ and R² include for example unsubstituted alkyl, alkoxyalkyl, hydroxyalkoxyalkyl, polyhydroxyalkyl, hydroxy poly alkyleneoxyalkyl, oxaalkyl, polyoxaalkyl and the like.

The term "acyl" as used herein includes both carboxylate and carbonate groups, thus, acyloxy substituted alkyl groups include for example alkylcarbonyloxy alkyl. In such groups any alkylene moieties preferably have carbon atom contents defined herein for alkyl groups.

Preferred substituted alkyl R¹ groups include those carrying one or more oxo groups, preferably straight-chained C₄₋₁₂ alkyl (e.g. C₈₋₁₀ alkyl) groups substituted by one, two or three (preferably two or three) oxo groups. Examples of such groups include 3,6-dioxa-1-octyl and 3,6,9-trioxa-1-decyl groups.

Particularly preferred substituted alkyl R¹ groups which may be present in compounds of formula I include C₁₋₆ alkyl, preferably C₁₋₄ alkyl, particularly preferably C₁ or C₄ alkyl (e.g. methyl) substituted (preferably terminally substituted) by an aryl group- Preferred aryl groups include phenyl, diphenyl and monocyclic 5-7 membered, e.g. 5 or 6-membered, heteroaromatics, especially phenyl and such groups may themselves optionally be substituted, for example by one or more (e.g. one or two) C₁₋₆ alkyl groups (preferably C₁₋₄ alkyl, e.g. methyl), alkoxy (e.g. methoxy), nitro, fluoro, chloro or trifluoromethyl groups. Suitable heteroaromatic groups include those containing at least one heteroatom selected from oxygen, sulphur and nitrogen. A preferred heteroaromatic group is pyridine.

Representative substituted alkyl groups R¹ and R² include alkoxymethyl, alkoxyethyl and alkoxypropyl groups or acyloxymethyl, acyloxyethyl and acyloxypropyl groups eg. pivaloyloxymethyl.

Preferred compounds for use according to the invention, include those wherein R¹ and/or R², preferably R¹ represents an unsubstituted alkyl group or an aryl substituted alkyl group (e.g. a benzyl group), in which the aryl group itself may also be substituted as described herein. Especially, R¹ is a C₁₋₁₆ alkyl group, e.g. a C₆ alkyl group or a benzyl group, both of which may optionally be substituted. Particularly preferable ALA esters are compounds in which R¹ is as described above, and at the N-terminal both R² groups are hydrogen atoms.

Underivatized ALA esters (ie. when R¹ is not a hydrogen atom) also form preferred photochemotherapeutic agents.

Particularly preferred for use in the invention are those compounds of formula I in which R¹ either represents an unsubstituted alkyl group (e.g. C₁₋₅ alkyl) or an alkyl group (e.g. C₁₋₂ alkyl) substituted by an aryl group (e.g. phenyl) and/or each R² represents a hydrogen atom.

Especially preferred compounds of formula I include 1-methylpentyl ALA ester, p-isopropylbenzyl ALA ester, p-methylbenzyl ALA ester, benzyl ALA ester, 2-phenylethyl ALA ester, hexyl ALA ester, cyclohexyl ALA ester, 4-methylpentyl ALA ester, p-[trifluoromethyl]benzyl ALA ester, p-[t-butyl]benzyl ALA ester, p-nitrobenzyl ALA ester, 1-ethylbutyl ALA ester, 2-methylpentyl ALA ester, 4-phenyl butyl ALA ester, p-fluorobenzyl ALA ester, 3,3-dimethyl-1-butyl ALA ester, 2-fluorobenzyl ALA ester, 2,3,4,5,6-pentafluorobenzyl ALA ester, 4-chlorobenzyl ALA ester, 2-methoxyethyl ALA ester, 3-nitrobenzyl ALA ester, 3,4-[di-chloro]benzyl ALA ester, 3,6-dioxa-1-octyl ALA ester, 3-fluorobenzyl ALA ester, 3,6,9-trioxa-1-decyl ALA ester, 3-pyridinyl-methyl ALA ester, 4-diphenyl-methyl ALA ester, 4-methoxy-benzyl ALA ester, 2-methylbenzyl ALA ester, benzyl-5-[(1-acetyloxyethoxy)-carbonyl]amino levulinate, and 3-methylbenzyl ALA ester.

Most preferred for use in the method of the invention are 5-ALA, 5-ALA methyl ester, 5-ALA hexyl ester and 5-ALA benzyl ester.

The above mentioned photochemotherapeutic compounds of the invention may be prepared using standard processes and procedures well-known in the art. For example, in the case of esterification of compounds this may involve protection and deprotection of appropriate groups such that only the required groups remain active and take part in the reaction under the conditions of the esterification. Appropriate methods for the preparation of ALA esters are described in WO96/28412.

As mentioned above, the ALA ester or derivative thereof as photochemotherapeutic agents used in compositions of the invention may take the form of pharmaceutically acceptable salts. Such salts preferably are acid addition salts with physiologically acceptable organic or inorganic acids. Suitable acids include, for example, hydrochloric, hydrobromic, sulphuric, phosphoric, acetic, lactic, citric, tartaric, succinic, maleic, fumaric and ascorbic acids. Hydrophobic salts may also conveniently be produced by for example precipitation. Appropriate salts include for example acetate, bromide, chloride, citrate, hydrochloride, maleate, mesylate, nitrate, phosphate, sulfate, tartrate, oleate, stearate, tosylate, calcium, meglumine, potassium and sodium salts. Procedures for salt formation are conventional in the art.

Mucoadhesive agents which may be used in compositions of the invention may be natural or synthetic, polyanionic, polycationic or neutral, water-soluble or water-insoluble, but are preferably large (e.g. having a molecular weight of 500 to 3000kDa, e.g. 1000 to 2000kDa), water-insoluble cross-linked (e.g. containing 0.05 to 2%, e.g. 0.75 to 1.5% cross-linker by weight of the total polymer, prior to any hydration), water-swellable polymers capable of forming hydrogen bonds. Preferably mucoadhesives according to the invention have a mucoadhesive force greater than 100, especially preferably greater than 120, particularly greater than 150, as assessed according to the method of Smart et al., 1984, J. Pharm. Pharmacol., 36, p295-299, expressed as a percent relative to a standard in vitro.

Appropriate mucoadhesives include, but are not limited to poly(carboxylic acid-containing) based polymers, such as poly(acrylic, maleic, itaconic, citraconic, hydroxyethyl methacrylic or methacrylic) acid which have strong hydrogen-bonding groups, or derivatives thereof such as salts and esters. Alternatively, cellulose derivatives may be used such as methyl cellulose, ethyl cellulose, methylethyl cellulose, hydroxymethyl cellulose, hydroxyethyl cellulose, hydroxypropyl cellulose, hydroxyethyl ethyl cellulose, carboxymethyl cellulose, hydroxypropylmethyl cellulose or cellulose esters or ethers or derivatives or salts thereof. Other naturally occurring or synthetic polymers may also be used such as gums, e.g. xanthan gum, guar gum, locust bean gum, tragacanth gums, karaya gum, ghatti gum, cholla gum, psillium seed gum and gum arabic; clays such as manomorillonite clays, e.g. Veegun, attapulgite clay; polysaccharides such as dextran, pectin, amylopectin, agar, mannan or polygalactonic acid or starches such as hydroxypropyl starch or carboxymethyl starch; lipophilic formulations containing polysaccharides, e.g. Orabase (Bristol Myers Squibb); carbohydrates such as polysubstituted with groups such as sulphate, phosphate, sulphonate or phosphonate, e.g. sucrose octasulphate; polypeptides such as casein, gluten, gelatin, fibrin glue; chitosan (lactate or glutamate) or carboxymethyl chitin; glycosaminoglycans such as hyaluronic acid; metals or water soluble salts of alginic acid such as sodium alginate or magnesium alginate; schleroglucan; adhesives containing bismuth oxide or aluminium oxide; atherocollagen; polyvinyl polymers such as polyvinyl *alcohols, polyvinylmethyl ethers, polyvinylpyrrolidone,* polycarboxylated vinyl polymers (such as polyacrylic acid as mentioned above); polysiloxanes; polyethers; polyethylene oxides and glycols; polyalkoxys and polyacrylamides and derivatives and salts thereof.

Mucoadhesives may also be used which bind to the epithelial cell layer lying below the mucous layer. This allows more specific and longer lasting adhesion due to the slower relative turnover of epithelial cells compared to mucous turnover (days rather than hours). Thus for example, receptor-mediated interactions may be achieved using plant or bacterial lectins, ie. (glyco)proteins of non-immune origin which bind to polysaccharides or glycoconjugates, which specifically bind to sugar moieties of the epithelial cell membrane. Also so-called "reverse" lectins of mammals in which receptors on the epithelial cell binds to sugars of the agent which is added, may be used. Other bioadhesives (e.g. adhesion or invasion factors, e.g. bacterial adhesins or invasins which bind to integrins) from bacteria or viruses may be used to allow selectively for particular tissues, phenotypes, disorders etc. by binding to only certain epithelial cells.

The above described polymeric mucoadhesives may also be cross-linked and may be in the form of copolymers. Preferably poly(acrylic acid) polymers (or copolymers, e.g. with di- or poly functional allyl ethers or acrylates to make the polymer insoluble), which have preferably been cross-linked, e.g. using a polyalkenyl polyether, are employed which have a high molecular weight and are thixotropic. Appropriate mucoadhesives having this form are available commercially (e.g. from Goodrich) as polycarbophil, e.g. Noveon AA-1, Carbomer (Carbopol), e.g. Carbopol EX165, EX214, 434, 910, 934, 934P, 940, 941, 951, 974P and 1342.

Some of the preferred mucoadhesives for use in the compositions of the invention thus include, polyacrylic hydrogels, chitosan, polyvinyl alcohol, hydroxypropyl cellulose, hydroxypropyl methyl cellulose, sodium alginate, scleroglucan, xanthan gum, pectin, orabase and polygalactonic acid.

The above mentioned mucoadhesives may be prepared using standard processes and procedures well-known in the art, although many are available commercially, e.g. from Goodrich, BDH, Hercules, Dow Chemical Co.

As mentioned above, in respect of the ALA esters or derivatives thereof as photochemotherapeutic agents, mucoadhesive derivatives or salts may be used. Appropriate salts are as described above in connection with the photochemotherapeutic agents. Mucoadhesive derivatives include chemically modified agents which retain mucoadhesive properties. Such derivatives include those which have been modified to include the photochemotherapeutic agent, for example salts such as ALA-methyl ester alginate or esters such as between ALA and hydroxypropyl cellulose may be formed.

By "pharmaceutically acceptable" or "physiologically acceptable" is meant that the ingredient must be compatible with other ingredients in the composition as well as physiologically acceptable to the recipient.

The ALA ester or derivative thereof as a photochemotherapeutic agent and the mucoadhesive may be combined into a composition or a product of the invention by any appropriate means depending on the condition to be treated or diagnosed. For example, depending on the mucoadhesive agent the composition may be formulated as a dry polymeric film, tablet or powder. In this case adhesion is likely to occur via hydration, as in the case of cellulose derivatives. Other formulations may be applied in a semi or fully hydrated state (e.g. hydrogels, such as poly(acrylic acid) polymers, hyaluronic acid and chitosan), e.g. by use of an aqueous solution.

The ALA ester or derivative thereof as a photochemotherapeutic agent may be attached or conjugated to, or associated with, or simply administered with, the mucoadhesive agent. It will be appreciated that one or more of each agent type may be used in any formulation. The formulation may be in any appropriate dosage form, for example as an emulsion or in liposomes, niosomes, microspheres, nanoparticles or the like in which the mucoadhesive is present on only a portion of the dosage form, e.g. the outer surface. The photochemotherapeutic agent may then be absorbed to, incorporated in or bound to these forms. The use of solutions, suspensions (particularly fine suspensions in which the components have a small particle size), gels and emulsions are preferred.

As mentioned above, the composition of the invention and their salts have valuable pharmacological properties, namely as photosensitizing agents for use on epithelial-lined, preferably mucosa-lined surfaces, which renders them useful as photochemotherapeutic agents at these sites.

As mentioned above, the present invention accordingly provides a pharmaceutical composition comprising an ALA ester or derivative thereof as a photochemotherapeutic compound and a mucoadhesive agent as described hereinbefore, or pharmaceutically acceptable salts thereof, optionally together with at least one pharmaceutical carrier or excipient.

The formulations comprise ALA ester or derivatives thereof as photochemotherapeutic agents selected from compounds of formula I above, and their derivatives, together with mucoadhesive agents selected from polyacrylic hydrogels, chitosan, polyvinyl alcohol, hydroxypropyl cellulose, hydroxypropyl methyl cellulose, sodium alginate, scleroglucan and xanthan gum. Particularly preferred are formulations containing ALA esters (such as for example C₁₋₁₆ alkyl e.g. hexyl ALA ester) with polyacrylic acid, chitosan, pectin, polygalactonic acid or orabase.

The abnormalities and disorders which may be treated according to the present invention include any malignant, pre-malignant and non-malignant abnormalities or disorders responsive to photochemotherapy eg. tumours or other growths, particularly basal cell carcinomas, dysplasia or other growths, and other diseases or infections eg. bacterial, viral or fungal infections, for example Herpes virus infections. The invention is particularly suited to the treatment of diseases, disorders or abnormalities where discrete lesions are formed to which the compositions may be directly applied (lesions is used here in a broad sense to include tumours and the like).

The body surfaces which may be treated according to the invention include epithelial-lined surfaces, preferably mucosa-lined surfaces which cover tissues which communicate directly or indirectly with the external environment, eg, the respiratory, gastrointestinal and genito-urinary tracts and ear canal. As used herein "epithelial-lined surfaces" refer to surfaces of the body wherein the uppermost layer comprises epithelial cells. These cells may or may not be covered with a substantially aqueous or gel layer, e.g. mucous.

Exemplary mucosa-lined surfaces thus include: (i) cornea and conjunctiva; (ii) the lining of the mouth, pharynx, oesophagus, stomach, intestines and intestinal appendages, rectum, and anal canal; (iii) the lining of the nasal passages, nasal sinuses, nasopharynx, trachea, bronchi, and bronchioles; (iv) the lining of the ureters, urinary bladder, and urethra; (v) the lining of the vagina, uterine cervix, and uterus; and (vi) the lining of the ear canal.

The compositions of the invention may be formulated in conventional manner with one or more physiologically acceptable carriers or excipients, according to techniques well known in the art. Where appropriate, in a preferred feature, compositions according to the invention are sterilized, e.g. by γ-irradiation, autoclaving or heat sterilization, before or after the addition of a carrier or excipient where that is present, to provide sterile formulations.

Compositions may be administered topically (e.g. by intestinal, buccal, sublingual, gingival, palatal, nasal, pulmonary, vaginal, rectal or ocular delivery), orally or parenterally. Topical compositions are preferred, and include gels, creams, ointments, sprays, lotions, salves, sticks, soaps, powders, tablets, films, pessaries, aerosols, drops, solutions and any of the other conventional pharmaceutical forms in the art.

Ointments, gels and creams may, for example, be formulated with an aqueous or oily base with the addition of suitable thickening and/or gelling agents. Lotions may be formulated with an aqueous or oily base and will, in general, also contain one or more emulsifying, dispersing, suspending, thickening or colouring agents. Powders may be formed with the aid of any suitable powder base. Drops and solutions may be formulated with an aqueous or non-aqueous base also comprising one or more dispersing, solubilising or suspending agents. Aerosol sprays are conveniently delivered from pressurised packs, with the use of a suitable propellant. It will be appreciated that depending on the mucoadhesive which is used in the formulation of the invention that some of the above mentioned properties may be intrinsically provided by the mucoadhesive of the formulation.

Alternatively, the compositions may be provided in a form adapted for oral or parenteral administration. Alternative pharmaceutical forms thus include plain or coated tablets, capsules, suspensions and solutions containing the active component optionally together with one or more inert conventional carriers and/or diluents, e.g. with corn starch, lactose, sucrose, microcrystalline cellulose, magnesium stearate, polyvinylpyrrolidone, citric acid, tartaric acid, water, water/ethanol, water/glycerol, water/sorbitol, water/polyethylene glycol, propylene glycol, stearyl alcohol, carboxymethylcellulose or fatty substances such as hard fat or suitable mixtures thereof, where these are not already present as mucoadhesives in the compositions of the invention.

The compositions may additionally include lubricating agents, wetting agents, emulsifying agents, suspending agents, preserving agents, sweetening agents, flavouring agents, adsorption enhancers, e.g. surface penetrating agents as mentioned below, and the like. The compositions of the invention may be formulated so as to provide quick, sustained or delayed release of the active ingredient after administration of the patient by employing procedures well known in the art. Solubilizing and/or stabilizing agents may also be used, e.g. cyclodextrins (CD) α, β, γ and HP-β cyclodextrin.

The concentration of the photochemotherapeutic ALA esters or derivatives thereof and mucoadhesive agents as described hereinbefore in the compositions of the invention, depends upon the nature of the compounds in the composition, the mode of administration, the condition to be treated and the patient and may be varied or adjusted according to choice. Generally however, concentration ranges for the photochemotherapeutic agent of 0.01 to 50%, e.g. 0.05 to 20%, e.g. 1-10% (w/w, of the final preparation for administration) are suitable. For therapeutic applications concentration ranges for the ALA esters or derivatives thereof as photochemotherapeutic agent of 0.1 to 50% are suitable, e.g. 0.2 to 30% (w/w).

The mucoadhesive agent may be present at a concentration of 0.05 to 50%, e.g. 0.1 to 25%, e.g. 0.2 to 10% (w/w) of the final composition.

The formulation is preferably administered topically, although administration may take place distally at the upper end of a tract containing the site of administration, e.g. oral administration for a gastric disorder. Conveniently, the mucoadhesive is selected to have improved adhesive properties at the site of interest, e.g. by using mucoadhesives which have altered adhesive properties at different pHs, or at different states of hydration or which are involved in specific receptor-type interactions to introduce specificity for a particular area.

Topical administration to inaccessible sites may be achieved by techniques known in the art, e.g. by the use of catheters or other appropriate drug delivery systems.

Following administration to the surface, the area treated is exposed to light to achieve the photochemotherapeutic effect. Methods for irradiation of different areas of the body, eg. by lamps or lasers are well known in the art (see for example Van den Bergh, Chemistry in Britain, May 1986 p. 430-439). For inaccessible regions this may conveniently be achieved using optical fibres.

The length of time following administration, at which the light exposure takes place will depend on the nature of the condition to be treated, the composition and the form of administration. This can generally be in the order of 0.5 to 48 hours, e.g. 1 to 10 hours.

The irradiation will in general be applied at a dose level of 40 to 200 Joules/cm², for example at 100 Joules/cm².

The wavelength of light used for irradiation may be selected to achieve a more efficacious photochemo-therapeutic effect. Conventionally, when porphyrins are used in photochemotherapy they are irradiated with light at about the absorption maximum of the porphyrin. Thus, for example in the case of the prior art use of ALA in photochemotherapy of skin cancer, wavelengths in the region 350-640 nm, preferably 610-635 nm were employed. However, by selecting a broad range of wavelengths for irradiation, extending beyond the absorption maximum of the porphyrin, the photosensitizing effect may be enhanced. Whilst not wishing to be bound by theory, this is thought to be due to the fact that when Pp, and other porphyrins, are exposed to light having wavelengths within its absorption spectrum, it is degraded into various photo-products including in particular photoprotoporphyrin (PPp). PPp is a chlorin and has a considerable photo-sensitizing effect; its absorption spectrum stretches out to longer wavelengths beyond the wavelengths at which Pp absorbs ie. up to almost 700 nm (Pp absorbs almost no light above 650 nm). Thus in conventional photochemotherapy, the wavelengths used do not excite PPp and hence do not obtain the benefit of its additional photosensitizing effect. Irradiation with wavelengths of light in the range 500-700 nm has been found to be particularly effective. It is particularly important to include the wavelengths 630 and 690 nm.

A further aspect of the invention thus provides the use of a composition as hereinbefore defined in the preparation of a medicament for photochemotherapeutic treatment or diagnosis of disorders or abnormalities of epithelial-lined surfaces of the human or animal body, preferably mucosa-lined surfaces. The treatment comprises comprising administering to the affected surfaces, a composition as hereinbefore defined, and exposing said surfaces to light, preferably to light in the wavelength region 300-800 nm, for example 500-700 nm.

The compositions for use in the invention may be formulated and/or administered with other photosensitizing agents, ALA esters may be administered for example with ALA or Photofrin®, or with more than one mucoadhesive, or with other active components which may enhance the photochemotherapeutic effect. As mentioned previously, chelating agents may be included. These may enhance the stability of the photochemotherapeutic agent or enhance accumulation of Pp; the chelation of iron by the chelating agents prevents its incorporation into Pp to form haem by the action of the enzyme ferrochelatase, thereby leading to a build-up of Pp. The photosensitizing effect is thus enhanced.

Aminopolycarboxylic acid chelating agents are particularly suitable for use in this regard, including any of the chelants described in the literature for metal detoxification or for the chelation of paramagnetic metal ions in magnetic resonance imaging contrast agents. Particular mention may be made of EDTA, CDTA (cyclohexane diamine tetraacetic acid), DTPA and DOTA and well known derivatives/analogues thereof. EDTA is preferred. To achieve the iron-chelating effect, desferrioxamine and other siderophores may also be used, e.g. in conjunction with aminopolycarboxylic acid chelating agents such as EDTA.

The chelating agent may conveniently be used at a concentration of 1 to 20% eg. 2 to 10% (w/w).

Additionally, it has been found that surface-penetration assisting agents and especially dialkylsuphoxides such as dimethylsulphoxide (DMSO) may have a beneficial effect in enhancing the photochemotherapeutic effect. This is described in detail in WO95/07077.

The surface-penetration assisting agent may be any of the membrane-penetration assisting agents described in the pharmaceutical literature e.g. chelators (e.g. EDTA), surfactants (e.g. sodium dodecyl sulphate), non-surfactants, bile salts (e.g. sodium deoxycholate, and fatty acids (e.g. oleic acid). Examples of appropriate surface penetration assisting agents include HPE -101 (available from Hisamitsu), DMSO and other dialkylsulphoxides, in particular n-decylmethylsulphoxide (NDMS), dimethylsulphacetamide, dimethylformamide (DMFA), dimethylacetamide, glycols, various pyrrolidone derivatives (Woodford et al., J. Toxicol. Cut. & Ocular Toxicology, 1986, 5: 167-177), and Azone® (Stoughton et al., *Drug Dpv. Ind. Pharm*. 1983, 9: 725-744), or mixtures thereof. DMSO is however preferred due to its anti-histamine and antiinflammatory activities and its stimulatory effect on the activity of the enzymes ALA-synthase and ALA-dehydrogenase.

The surface penetration agent may conveniently be provided in a concentration range of 0.2 to 50% (w/w), eg about 10% (w/w).

The compositions of the invention are used according to the invention may additionally be formulated and/or administered with other agents, to improve the efficacy of PDT. Thus, for example, when treating tumours for example, angiogenesis inhibitors (anti-angiogenic drugs) which have been found to be useful for treating tumours (O'Reilly et al., Nature Medicine, 2, p689-692, 1996; Yamamoto et al., Anticancer Research, 14, p1-4, 1994; and Brooks et al., J. Clin. Invest., 96, p1815-1822, 1995) may be used together with compositions of the invention in PDT to further damage the vascular system of the tumour. Angiogenesis inhibitors which may be used include TNP-470 (AGM-1470, a synthetic analogue of a fungal secretion product called fumagillin; Takeda Chemical Industries Ltd., Osaka, Japan), angiostatin (Surgical Research Lab. at Children's Hospital Medical Center of Harvard Medical School) and integrin αᵥβ₃ antagonists (e.g. monoclonal antibody to integrin αᵥβ₃, The Scripps Research Institute, LaJolla, CA).

Alternatively, or additionally, immunotherapy agents (e.g. antibodies or effectors such as macrophage activating factor) or chemotherapy agents may be used to improve PDT according to the invention. Administration of these supplementary agents should be performed in terms of route, concentration and formulation, according to known methods for using these agents. These additional agents may be administered before, after or during PDT, depending on their function. For example, angiogenesis inhibitors may be added 5 to 10 days after PDT to prevent tumour regrowth.

Other anti-cancer agents may similarly be used in combination with a composition of the invention, either as part of the formulation or as a separate treatment to be administered simultaneously, separately or sequentially.

Glucose has also been found to assist PDT when applied either topically or systemically. Although not wishing to be bound by theory, it appears that administration of glucose results in a lowering of pH which increase the hydrophobic properties of protoporphyrins, e.g. ALA, such that they can penetrate cells more easily. When topical administration is contemplated, conveniently the formulation, e.g. a cream, may contain 0.01% to 10% glucose (w/w).

According to the condition being treated, and the nature of the composition, the composition for use in the invention may be co-administered with such other optional agents, for example in a single composition or they may be administered sequentially or separately. Indeed, in many cases a particularly beneficial photochemotherapeutic effect may be obtained by pre-treatment with the surface-penetration assisting agent in a separate step, prior to administration of the compounds for use in the invention.

Furthermore, in some situations a pre-treatment with the surface-penetration assisting agent, followed by administration of the photochemotherapeutic agent in conjunction with the surface-penetration assisting agent may be beneficial. When a surface-penetration assisting agent is used in pre-treatment this may be used at high concentrations, e.g. up to 100% (w/w). If such a pre-treatment step is employed, the photochemotherapeutic agent may subsequently be administered up to several hours following pre-treatment eg. at an interval of 5-60 minutes following pre-treatment.

In a further aspect, the present invention also provides a kit for use in photochemotherapy or diagnosis of disorders or abnormalities of epithelial-lined, preferably mucosa-lined surfaces of the body comprising:
a) a first container containing an ALA ester or a derivative thereof as a photochemotherapeutic agent as described hereinbefore or a pharmaceutically acceptable salt thereof,
b) a second container containing a mucoadhesive agent as described hereinbefore or a pharmaceutically acceptable salt thereof,
c) a third container containing at least one surface penetration assisting agent; and optionally
d) one or more chelating agents contained within either said first, second or third container or in a fourth container.

It will be appreciated that the method of therapy using compounds as described hereinbefore inevitably involves the fluorescence of the disorder or abnormality to be treated. Whilst the intensity of this fluorescence may be used to eliminate abnormal cells, the localization of the fluorescence may be used to visualize the size, extent and situation of the abnormality or disorder.

The abnormality or disorder thus identified or confirmed at the site of investigation may then be treated through alternative therapeutic techniques e.g. surgical or chemical treatment, or by the method of therapy of the invention by continued build up of fluorescence or through further application of compositions of the invention at the appropriate site. It will be appreciated that diagnostic techniques may require lower levels of fluorescence for visualization than used in therapeutic treatments. Thus, generally, concentration ranges of 0.2 to 30% e.g. 1-5% (w/w), are suitable. Sites, methods and modes of administration have been considered before with regard to the therapeutic uses and are applicable also to diagnostic uses described here.

The compositions for use in the invention may also be used for in vitro diagnostic techniques, for example for examination of the cells contained in body fluids. The higher fluorescence associated with non-normal tissue may conveniently be indicative of an abnormality or disorder. This method is highly sensitive and may be used for early detection of abnormalities or disorders, for example bladder or lung carcinoma by examination of the epithelial cells in urine or sputum samples, respectively. Other useful body fluids which may be used for diagnosis in addition to urine and sputum include blood, semen, tears, spinal fluid etc. Tissue samples or preparations may also be evaluated, for example biopsy tissue. The technique may particularly successfully be performed using mucoadhesives which bind epithelial cells underlying the mucosa rather than binding the mucosa itself. The present invention thus extends to the use of compositions of the invention for diagnosis according to the aforementioned methods for photochemotherapy, and products and kits for performing said diagnosis.

A further aspect of the invention relates to a method of in vitro diagnosis, of abnormalities or disorders of epithelial-, preferably mucosa-lined surfaces, by assaying a sample of body fluid or tissue of a patient, said method comprising at least the following steps:
i) admixing said body fluid or tissue with a composition as described hereinbefore,
ii) exposing said mixture to light,
iii) ascertaining the level of fluorescence, and
iv) comparing the level of fluorescence to control levels.

The invention will now be described in more detail in the following non-limiting Examples in which the Figures referred to are as follows:

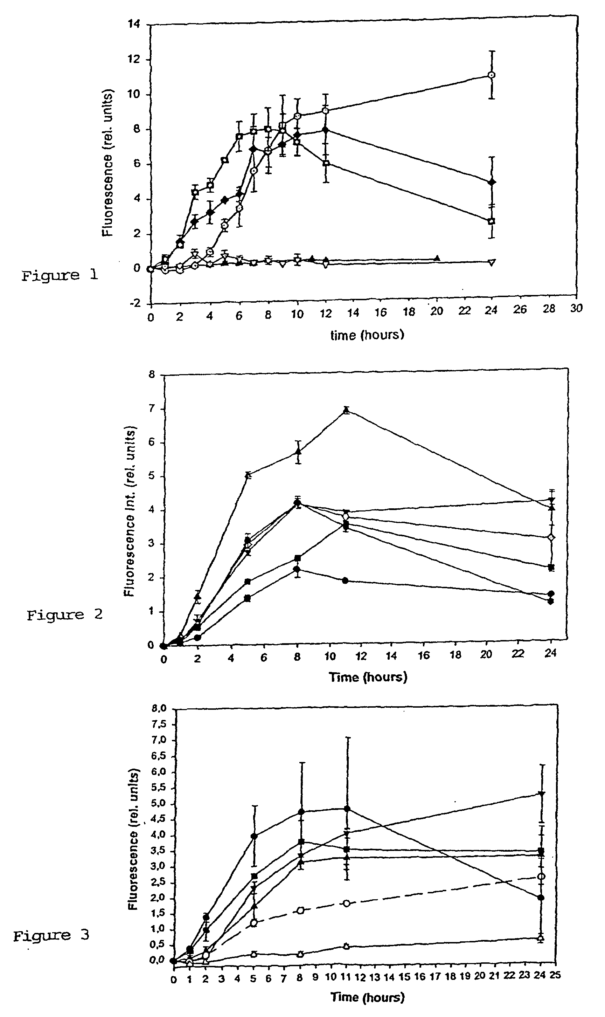

Figure 1 shows skin fluorescence after topical administration of various concentrations of methyl 5-aminolevulinate-HCl (P-1202) in Chitosan CL110 in which the filled triangles, filled squares, shaded hexagons, shaded inverted triangles and partially filled squares denote mucoadhesive formulations 1 to 5 respectively;
Figure 2 shows skin fluorescence after topical administration of hexyl 5-aminolevulinate-HCl with various mucoadhesive agents in which the filled squares, shaded triangles, filled inverted triangles, shaded squares, filled hexagons and filled circles denote mucoadhesive formulations 6 to 11 respectively; and
Figure 3 shows skin fluorescence after topical administration of hexyl 5-aminolevulinate-HCl at 5 or 10% with various mucoadhesive agents in which the filled squares, filled triangles, shaded inverted triangles, open circles, open triangles and filled circles denote mucoadhesive formulations 12 to 17 respectively, and in which the bars in this and preceding figures shows'the standard error of the mean.

### Example 1

### Mucoadhesive Gel

Hydroxymethyl cellulose (1.0g) is dissolved in water (100ml) at 70°C. The mixture is cooled and 5-ALA hexyl ester.HCl (prepared according to WO96/28412) (300mg) is dissolved in the mixture at 30°C. The gel is aliquoted in 2ml tubes. Each tube contains 2ml 0.3% 5-ALA hexyl ester in a mucoadhesive gel for single use in the mouth and/or oesophagus.

### Example 2

### Methods

Balb/c athymic nude mice, weighting 20-22g, obtained from the Department of Laboratory Animals, DNR were used in the study.

Each mouse received 0.05-0.1g of formulation topically applied at the right flank of the body, evenly distributed and covered with a dressing (*Opsite Flexigrid;* Smith and Nephew Medical Ltd., Hull, England). The fiber point measuring device consisted of a bundle of optical fibers connected to a spectrofluorimeter which produced the excitation light of 407 nm. The excitation light was led through half of the fibers to the mouse skin. The resulting emission fluorescence spectrum (550-750 nm) was collected and led through the remaining fibers into a photomultiplier for quantification.

After administration of the formulations, the fluorescence spectrum from the skin was measured by the fiber-optical method at different intervals of time after administration.

The skin of the nude mice was used in the Example below and is believed to serve as a good model for superficial mucosa for certain internal hollow organs since, histologically, the skin is similar to the mucosas of the oral cavity, oesophagus, cervix etc.

The formulations tested comprised methyl or hexyl ALA esters with Chitosan (Protosan CL 110, from Norsk Hydro, Pronova Medical Products, Oslo), Orabase™ paste (Bristol Myers Squibb), pectin (GENU Type: LM-104 AS-Z or X-8902, Copenhagen pectin; or Classic AU201 or CU501, both Herbstreith & Fox KG), chitosan glutamate (PMC Biopoymer 905-360-01), chitosan lactate (FMC Biopolymer 810-361-05), polyacrylic acid (Fluka), polygalactonic acid (Sigma) or methylhydropropylcellulosum 4000 (NMD, Oslo).

The formulations were used as follows in which m-ALA is methyl 5-aminolevulinate HCl and h-ALA is hexyl 5-aminolevulinate HCl:

| **Form. No.** | **Ingredients** | **Content of m/h-ALA** **% (w/w)** |
|---|---|---|
| Muco-1 | 0.15g m-ALA | 1 |
| | 2.25g Protosan CL 110 | |
| | Unguentum Merck ad 15g | |
| Muco-2 | 1.5g m-ALA | 10 |
| | 2.25g Protosan CL110 | |
| | Ung. Merck ad 15g | |
| Muco-3 | 0.3g m-ALA | 2 |
| | 1.5g Protosan CL110 | |
| | Ung. Merck ad 15g | |
| Muco-4 | 0.15g m-ALA | 1 |
| | 0.75g Protosan CL110 | |
| | Ung. Merck ad 15g | |
| Muco-5 | 1.5g m-ALA | 10 |
| | 0.75g Protosan CL110 | |
| | Ung. Merck ad 15g | |
| Muco-6 | 1.5g h-ALA | 10 |
| | 2.0g liquid paraffin | |
| | Orabase paste ad 15 g | |
| Muco-7 | 1.5g h-ALA | 10 |
| | 3.0g aqua pur. | |
| | 0.5g pectin (GENU Type: LM-104 AS-Z) | |
| | Ung. Merck ad 15 g | |
| Muco-8 | 1.5g h-ALA | 10 |
| | 3.0g aqua pur. | |
| | 1.5g chitosan glutamate (PMC Biopolymer 905-360-01) | |
| | Ung. Merck ad 15g | |
| Muco-9 | 1.5g h-ALA | 10 |
| | 1.5g chitosan lactate (FMC Biopolymer 810-361-05) | |
| | 12g cream* | |
| Muco-10 | 1.5g h-ALA | 10 |
| | 1.5g polyacrylic acid (Fluka) Ung. Merck ad 15 g | |
| Muco-11 | 1.5g h-ALA | 10 |
| | 1.5g methylhydroxypropyl | |
| | cellulosum 4000 (NMD, Oslo) | |
| | 7.0g Ung. Merck | |
| | 5.0g Orabase paste | |
| Muco-12 | 1.5g h-ALA | 10 |
| | 0.5g pectin (GENU LM-104 AS-Z) | |
| | 3.0g aqua pur. | |
| | 1.0g cream (see Muco-9) | |
| Muco-13 | 1.5g h-ALA | 10 |
| | 0.5g pectin (Classic CU 501 Herbstreith & Fox KG) | |
| | 3.0g aqua pur. | |
| | 10 Ung. Merck | |
| Muco-14 | 1.5g h-ALA | 10 |
| | 1.0g polygalactonic acid (Sigma) | |
| | 3.0g aqua pur. | |
| | 9.5g Ung.Merck | |
| Muco-15 | 1.5g h-ALA | 10 |
| | 1.0g pectin (Classic CU 501 Herbstreith & Fox KG) | |
| | 3.0g aqua pur. | |
| | 9.5g Ung.Merck | |
| Muco-16 | 1.5g h-ALA | 10 |
| | 0.5g pectin (GENU X-8902, Copenhagen pectin) | |
| | 13g ointment** | |
| Muco-17 | 0.75g h-ALA | 5 |
| | 0.5g pectin (GENU LM-104 AS-Z Copenhagen pectin) | |
| | 3.0g aqua pur. | |
| | Ung. Merck ad 15g | |

| | | |
|---|---|---|
| * cream = 2.0g urea, 2.5g sorbitan oleate, 0.5g Polysorbate 80, 47g Vaseline white, 48g aqua pur. | | |
| **ointment contained 10% zinc oxide (Sinksalve 10%, Nycomed Pharma ASA, Oslo) | | |

### Results

The results are shown in Figures 1 to 3.

### Example 3

### Study

Methylene blue (0.1 wt.%) was added to each of the formulations Muco-6, Muco-10, Muco-13 and Muco-14 described in Example 2. Uguentum Merck containing the same concentration of methylene blue was used as a control.

In turn, each formulation was applied to the mouth, tongue and between the teeth of a man. Visual inspection of the mouth to determine the extent of blue staining after about one hour was used to determine the extent of mucoadhesion. All formulations tested (i.e. Muco-6, Muco-10, Muco-13 and Muco-14) showed improved mucoadhesive properties over the control. Muco-6 demonstrated particularly good mucoadhesion.

## Claims

1. A pharmaceutical composition comprising a photochemotherapeutic agent and a mucoadhesive agent, wherein said photochemotherapeutic agent is an ALA ester or derivative thereof, optionally together with at least one pharmaceutically acceptable carrier or excipient.

2. A composition as claimed in claim 1 which further comprises at least one surface penetration assisting agent and/or one or more chelating agents.

3. A composition as claimed in claim 1 or claim 2 wherein said photochemotherapeutic agent is present at a concentration of from 0.01 to 50% by weight.

4. A composition as claimed in any one of claims 1 to 3 wherein said mucoadhesive agent is present at a concentration of from 0.05 to 50% by weight.

5. A composition as claimed in any one of claims 1 to 4 wherein said agent is a compound of formula I:
R² ₂N-CH₂COCH₂-CH₂CO-OR¹ (I)
(wherein
R¹ and R² each independently represents a hydrogen atom or an optionally substituted straight-chained, branched or cyclic alkyl group which may optionally be interrupted by one or more -O-, -NR³-, -S- or -PR³- groups, with the proviso that R' is other than hydrogen; and
R³ is a hydrogen atom or a C₁₋₆ alkyl group) or a pharmaceutically acceptable salt thereof.

6. A composition as claimed in claim 5 wherein in formula I, R¹ either represents an unsubstituted alkyl group (e.g. C₁₋₆ alkyl) or an alkyl group (e.g. C₁₋₂ alkyl) substituted by an aryl group (e.g. phenyl) and/or each R² represents a hydrogen atom.

7. A composition as claimed in claim 5 or claim 6 wherein R¹ represents a C₁₋₁₆ alkyl group.

8. A composition as claimed in claim 7 wherein R¹ is a straight-chained saturated hydrocarbon selected from hexyl, heptyl and octyl.

9. A composition as claimed in claim 5 wherein in formula I, R¹ represents a substituted benzyl group and each R² represents a hydrogen atom.

10. A composition as claimed in claim 9 wherein said photochemotherapeutic agent is p-isopropylbenzyl ALA ester, p-methylbenzyl ALA ester, p-[tri-fluoromethyl]-benzyl ALA ester, p-[t-butyl]benzyl ALA ester, p-nitrobenzyl ALA ester, p-fluorobenzyl ALA ester, 2-fluorobenzyl ALA ester, 2,3,4,5,5-pentafluorobenzyl ALA ester, 4-chlorobenzyl ALA ester, 3-nitrobenzyl ALA ester, 3-fluorobenzyl ALA ester, 4-methoxy-benzyl ALA ester, 2-methylbenzyl ALA ester or 3-methylbenzyl ALA ester.

11. A composition as claimed in any one of claims 1 to 4 wherein the ALA ester is 5-ALA methyl ester, 5-ALA hexyl ester or 5-ALA benzyl ester.

12. A composition as claimed in any preceding claim wherein said mucoadhesive agent is a water-swellable polymer capable of forming hydrogen bonds.

13. A composition as claimed in claim 12 wherein said polymer is selected from poly(carboxylic acid-containing) based polymers, cellulose derivatives or cellulose esters or ethers, gums, clays, polysaccharides, starches, lipophilic formulations containing polysaccharides, carbohydrates optionally substituted with sulphate, phosphate, sulphonate or phosphonate groups, polypeptides, chitosan (lactate or glutamate) or carboxymethyl chitin, glycosaminoglycans, metals or water soluble salts of alginic acid, schleroglucan, adhesives containing bismuth oxide or aluminium oxide, atherocollagen, polyvinyl polymers, polysiloxanes, polyethers, polyethylene oxides and glycols, polyalkoxys and polyacrylamides, and derivatives and salts thereof.

14. A composition as claimed in claim 13 wherein said mucoadhesive is selected from poly(acrylic, maleic, itaconic, citraconic, hydroxyethyl methacrylic or methacrylic) acid, methyl cellulose, ethyl cellulose, methylethyl cellulose, hydroxymethyl cellulose, hydroxyethyl cellulose, hydroxypropyl cellulose, hydroxyethyl ethyl cellulose, carboxymethyl cellulose, hydroxypropylmethyl cellulose, xanthan gum, guar gum, locust bean gum, tragacanth gums, karaya gum, ghatti gum, cholla gum, psillium seed gum, gum arabic, Veegun, attapulgite clay, dextran, pectin, amylopectin, agar, mannan, polygalactonic acid, hydroxypropyl starch, carboxymethyl starch, Orabase™, sucrose octasulphate, casein, gluten, gelatin, fibrin glue, hyaluronic acid, sodium alginate or magnesium alginate, polyvinyl alcohols, polyvinylmethyl ethers, polyvinylpyrrolidone, polycarboxylated vinyl polymers, and derivatives and salts thereof.

15. A composition as claimed in claim 1 together with a mucoadhesive agent selected from polyacrylic hydrogels, chitosan, polyvinyl alcohol, hydroxypropyl cellulose, hydroxypropyl methyl cellulose, sodium alginate, scleroglucan, xanthan gum, pectin, polygalactonic acid and orabase.

16. A composition as claimed in any one of claims 1 to 15 wherein said composition is in the form of an ointment, gel or cream with an aqueous base.

17. A composition as defined in any preceding claim for use as a medicament.

18. A composition as claimed in claim 17 for use in the treatment or diagnosis of disorders or abnormalities of epithelial-lined surfaces.

19. A composition as claimed in claim 18 wherein said epithelial-lined surfaces are mucosa-lined surfaces.

20. A composition as claimed in claim 19 wherein said mucosa-lined surfaces are the lining of the mouth, pharynx, oesophagus, stomach, intestines and intestinal appendages, rectum, anal canal, nasal passages, nasal sinuses, nasopharynx, trachea, bronchi, bronchioles, ureter, urinary bladder, urethra, vagina, uterine cervix or uterus.

21. A composition as claimed in any one of claims 18 to 20 for use in treating malignant, pre-malignant and non-malignant abnormalities or disorders responsive to photochemotherapy, preferably tumours or other growths, particularly basal cell carcinomas, dysplasia or other growths, and other diseases or infections, e.g. bacterial, viral or fungal infections, for example Herpes virus infections.

22. Use of a photochemotherapeutic agent as defined in any one of claims 1 or 5 to 11 together with a mucoadhesive as defined in any one of claims 1 or 12 to 15, optionally together with at least one surface penetration assisting agent and optionally with one or more chelating agents in the preparation of a composition for the treatment or diagnosis of disorders or abnormalities as defined in claim 18 or 21 of epithelial-lined surfaces as defined in any one of claims 18 to 20.

23. A product comprising a photochemotherapeutic agent as defined in any one of claims 1 or 5 to 11 and a mucoadhesive as defined in any one of claims 1 or 12 to 15, optionally together with at least one surface penetration assisting agent and optionally one or more chelating agents as a combined preparation for simultaneous, separate or sequential use in treating or diagnosing disorders or abnormalities as defined in claim 18 or 21 of epithelial-lined surfaces as defined in any one of claims 18 to 20.

24. Use of a photochemotherapeutic agent as defined in any one of claims 1 or 5 to 11 and a mucoadhesive as defined in any one of claims 1 or 12 to 15, optionally together with at least one surface penetration assisting agent and optionally one or more chelating agents in the preparation of a product for simultaneous, separate or sequential use in treating or diagnosing disorders or abnormalities as defined in claim 18 or 21 of epithelial-lined surfaces as defined in any one of claims 18 to 20.

25. A kit for use in photochemotherapy or diagnosis of disorders or abnormalities of epithelial-lined, preferably as defined in claim 19 or 20, surfaces of the body comprising:
a) a first container containing a photochemotherapeutic agent as defined in any one of claims 1 or 5 to 11, or a pharmaceutically acceptable salt thereof,
b) a second container containing a mucoadhesive agent as defined in any one of claims 1 or 12 to 15, or a pharmaceutically acceptable salt thereof,
c) a third container containing at least one surface penetration assisting agent; and optionally
d) one or more chelating agents contained within either said first, second or third container or in a fourth container.

26. A method of in vitro diagnosis of abnormalities or disorders of epithelial-lined surfaces preferably as defined in claim 19 or 20, by assaying a sample of body fluid or tissue of a patient, said method comprising at least the following steps:
i) admixing said body fluid or tissue with a composition as defined in any one of claims 1 to 16,
ii) exposing said mixture to light,
iii) ascertaining the level of fluorescence, and
iv) comparing the level of fluorescence to control levels.

## Patentansprüche

1. Pharmazeutische Zubereitung, umfassend ein Photochemotherapeutikum und ein Mucoadhäsivum, wobei es sich bei dem Photochemotherapeutikum um einen ALA-Ester oder ein Derivat davon handelt, gegebenenfalls zusammen mit wenigstens einem pharmazeutisch verträglichen Träger oder Exzipienten.

2. Zubereitung nach Anspruch 1, außerdem umfassend wenigstens einen Oberflächenpenetrations-Hilfsstoff und/oder einen oder mehrere Chelatbildner.

3. Zubereitung nach Anspruch 1 oder 2, wobei das Photochemotherapeutikum in einer Konzentration von 0,01 bis 50 Gew.-% vorliegt.

4. Zubereitung nach einem der Ansprüche 1 bis 3, wobei das Mucoadhäsivum in einer Konzentration von 0,05 bis 50 Gew.-% vorliegt.

5. Zubereitung nach einem der Ansprüche 1 bis 4, wobei es sich bei dem Therapeutikum um eine Verbindung der Formel I handelt:
R² ₂N-CH₂COCH₂-CH₂CO-OR¹ I
(worin
R¹ und R² unabhängig voneinander für ein Wasserstoffatom oder eine gegebenenfalls substituierte geradkettige, verzweigte oder cyclische Alkylgruppe stehen, die gegebenenfalls durch eine oder mehrere Gruppen -O-, -NR³-, -S- oder -PR³- unterbrochen ist, mit der Maßgabe, dass R¹ von Wasserstoff verschieden ist; und
R³ für ein Wasserstoffatom oder eine C₁₋₆-Alkylgruppe steht) oder ein pharmazeutisch verträgliches Salz davon.

6. Zubereitung nach Anspruch 5, wobei in der Formel I R¹ entweder für eine unsubstituierte Alkylgruppe (z.B. C₁₋₆-Alkyl) oder eine mit einer Arylgruppe (z.B. Phenyl) substituierte Alkylgruppe (z.B. C₁₋₂-Alkyl) steht und/oder jedes R² für ein Wasserstoffatom steht.

7. Zubereitung nach Anspruch 5 oder 6, wobei R¹ für eine C₁₋₁₆-Alkylgruppe steht.

8. Zubereitung nach Anspruch 7, wobei R¹ für einen geradkettigen gesättigten Kohlenwasserstoff steht, ausgewählt unter Hexyl, Heptyl und Octyl.

9. Zubereitung nach Anspruch 5, wobei in der Formel I R¹ für eine substituierte Benzylgruppe steht und jedes R² für ein Wasserstoffatom steht.

10. Zubereitung nach Anspruch 9, wobei es sich bei dem Photochemotherapeutikum um p-Isopropylbenzyl-ALA-ester, p-Methylbenzyl-ALA-ester, p-[Trifluormethyl]-benzyl-ALA-ester, p-[t-Butyl]benzyl-ALA-ester, p-Nitrobenzyl-ALAester, p-Fluorbenzyl-ALA-ester, 2-Fluorbenzyl-ALA-ester, 2,3,4,5,6-Pentafluorbenzyl-ALA-ester, 4-Chlorbenzyl-ALA-ester, 3-Nitrobenzyl-ALA-ester, 3-Fluorbenzyl-ALA-ester, 4-Methoxy-benzyl-ALA-ester, 2-Methylbenzyl-ALA-ester oder 3-Methylbenzyl-ALA-ester handelt.

11. Zubereitung nach einem der Ansprüche 1 bis 4, wobei es sich bei dem ALA-Ester um 5-ALA-Methylester, 5-ALA-Hexylester oder 5-ALA-Benzylester handelt.

12. Zubereitung nach einem der vorhergehenden Ansprüche, wobei das Mucoadhäsivum ein zur Ausbildung von Wasserstoffbrücken fähiges wasserquellbares Polymer ist.

13. Zubereitung nach Anspruch 12, wobei das Polymer ausgewählt ist unter Polymeren auf Basis von Polycarbonsäuren, Cellulosederivaten oder Celluloseestem oder -ethern, Gummis, Ton, Polysacchariden, Stärken, polysaccharidhaltigen lipophilen Formulierungen, gegebenenfalls mit Sulfat-, Phosphat-, Sulfonat- oder Phosphonatgruppen substituierten Kohlenhydraten, Polypeptiden, Chitosan (Lactat oder Glutamat) oder Carboxymethylchitin, Glycosaminoglycanen, Metall- oder wasserlöslichen Salzen von Alginsäure, Scleroglucan, Bismuthoxid- oder Aluminiumoxid-haltigen Klebstoffen, Atherocollagen, Polyvinylpolymeren, Polysiloxanen, Polyethern, Polyethylenoxiden und -glykolen, Polyalkoxiden und Polyacrylamiden, und Derivaten und Salzen davon.

14. Zubereitung nach Anspruch 13, wobei das Mucoadhäsivum ausgewählt ist unter Polyacryl-, -malein-, -itacon-, -citracon-, -hydroxyethylmethacryl- oder methacrylsäure, Methylcellulose, Ethylcellulose, Methylethylcellulose, Hydroxymethylcellulose, Hydroxyethylcellulose, Hydroxypropylcellulose, Hydroxyethylethylcellulose, Carboxymethylcellulose, Hydroxypropylmethylcellulose, Xanthangummi, Guargummi, Johannisbrotgummi, Traganthgummis, Karaya-Gummi, Ghatti-Gummi, Chollagummi, Flohsamengummi, Gummi arabicum, Veegun, Attapulgit-Ton, Dextran, Pektin, Amylopektin, Agar, Mannan, Polygalactonsäure, Hydroxypropylstärke, Carboxymethylstärke, Orabase™, Sucroseoctasulphat, Kasein, Gluten, Gelatine, Fibrinkleber, Hyaluronsäure, Natriumalginat oder Magnesiumalginat, Polyvinylalkohole, Polyvinylmethylether, Polyvinylpyrrolidon, polycarboxylierten Vinylpolymeren und Derivaten und Salzen davon.

15. Zubereitung nach Anspruch 1, zusammen mit einem Mucoadhäsivum, das ausgewählt ist unter Polyacrylhydrogelen, Chitosan, Polyvinylalkohol, Hydroxypropylcellulose, Hydroxypropylmethylcellulose, Natriumalginat, Scleroglucan, Xanthangummi, Pektin, Polygalactonsäure und Orabase.

16. Zubereitung nach einem der Ansprüche 1 bis 15, wobei die Zubereitung in Form einer Salbe, eines Gels oder einer Creme mit einer wässrigen Grundlage vorliegt.

17. Zubereitung gemäß Definition in einem der vorhergehenden Ansprüche zur Verwendung als Medikarnent.

18. Zubereitung nach Anspruch 17 zur Verwendung zur Behandlung oder Diagnose von Krankheiten oder Störungen von epithelbedeckten Oberflächen.

19. Zubereitung nach Anspruch 18, wobei die epithelbedeckten Oberflächen schleimhautbedeckte Oberflächen sind.

20. Zubereitung nach Anspruch 19, wobei die schleimhautbedeckten Oberflächen die Mundschleimhaut, die Rachenhöhle, Speiseröhre, der Magen, Darm und Darmanhänge, das Rektum, der Analkanal, die Nasenwege, Nasenhöhlen, der Nasopharynx, die Luftröhre, Bronchien, Bronchiolen, der Harnleiter, die Hamblase, die Harnröhre, Vagina, der Gebärmutterhals oder Uterus sind.

21. Zubereitung nach einem der Ansprüche 18 bis 20 zur Verwendung zur Behandlung bösartiger, prämaligner und nichtmaligner Störungen oder Erkrankungen, die auf Photochemotherapie reagieren, vorzugsweise von Tumoren oder anderen Geschwulsten, insbesondere Basalzellkarzinomen, Dysplasie oder anderen Geschwulsten, und anderen Erkrankungen oder Karzinomen, z.B. bakterieller, viraler oder Pilzinfektionen, z.B. von Herpesvirus-Infektionen.

22. Verwendung eines Photochemotherapeutikums gemäß Definition in einem der Ansprüche 1 oder 5 bis 11 zusammen mit einem Mucoadhäsivum gemäß Definition in einem der Ansprüche 1 oder 12 bis 15, gegebenenfalls zusammen mit wenigstens einem Oberflächenpenetrations-Hilfsstoff und gegebenenfalls mit einem oder mehreren Chelatbildnern zur Herstellung einer Zubereitung zur Behandlung oder Diagnose von Erkrankungen oder Störungen gemäß Definition in Anspruch 18 oder 21 von epithelbedeckten Oberflächen gemäß Definition in einem der Ansprüche 18 bis 20.

23. Erzeugnis, umfassend ein Photochemotherapeutikum gemäß Definition in einem der Ansprüche 1 oder 5 bis 11 und ein Mucoadhäsivum gemäß Definition in einem der Ansprüche 1 oder 12 bis 15, gegebenenfalls zusammen mit wenigstens einem Oberflächenpenetrations-Hilfsstoff und gegebenenfalls einem oder mehreren Chelatbildnern in Form einer kombinierten Zubereitung zur gleichzeitigen, getrennten oder aufeinanderfolgenden Verwendung zur Behandlung oder Diagnose von Erkrankungen oder Störungen gemäß Definition in Anspruch 18 oder 21 von epithelbedeckten Oberflächen gemäß Definition in einem der Ansprüche 18 bis 20.

24. Verwendung eines Photochemotherapeutikums gemäß Definition in einem der Ansprüche 1 oder 5 bis 11 und eines Mucoadhäsivums gemäß Definition in einem der Ansprüche 1 oder 12 bis 15, gegebenenfalls zusammen mit einem Oberflächenpenetrations-Hilfsstoff und gegebenenfalls einem oder mehreren Chelatbildnem zur Herstellung eines Erzeugnisses zur gleichzeitigen, getrennten oder aufeinanderfolgenden Verwendung zur Behandlung oder Diagnose von Erkrankungen oder Störungen gemäß Definition in Anspruch 18 oder 21 von epithelbedeckten Oberflächen gemäß Definition in einem der Ansprüche 18 bis 20.

25. Kit zur Anwendung in der Photochemotherapie oder Diagnose von Erkrankungen oder Störungen epithelbedeckter Oberflächen des Körpers, vorzugsweise solcher gemäß Definition in Anspruch 19 oder 20, umfassend:
a) einen ersten Behälter mit einem Photochemotherapeutikum gemäß Definition in einem der Ansprüche 1 oder 5 bis 11 oder einem pharmazeutisch verträglichen Salz davon,
b) einen zweiten Behälter mit einem Mucoadhäsivum gemäß Definition in einem der Ansprüche 1 oder 12 bis 15 oder einem pharmazeutisch verträglichen Salz davon,
c) einen dritten Behälter mit wenigstens einem Oberflächenpenetrations-Hilfsstoff; und gegebenenfalls
d) einen oder mehrere Chelatbildner, die entweder in dem ersten, zweiten oder dritten Behälter oder einem vierten Behälter vorgehalten werden.

26. Verfahren zur *in vitro*-Diagnose von Störungen oder Erkrankungen von epithelbedeckten Oberflächen, vorzugsweise solcher gemäß Definition in Anspruch 19 oder 20, indem man eine Probe einer Körperflüssigkeit oder eines Gewebes eines Patienten untersucht, wobei man zumindest:
i) die Körperflüssigkeit oder das Gewebe mit einer Zubereitung gemäß Definition in einem der Ansprüche 1 bis 16 mischt,
ii) Licht auf das Gemisch einwirken lässt,
iii) den Fluoreszenzgrad bestimmt, und
iv) den Fluoreszenzgrad mit Kontrollgraden vergleicht.

## Revendications

1. Composition pharmaceutique comprenant un agent photochimiothérapeutique et un agent qui adhère à la muqueuse, dans laquelle ledit agent photochimiothérapeutique est un ester d'ALA ou un dérivé de cet ester, facultativement conjointement avec au moins un excipient ou un véhicule pharmaceutiquement acceptable.

2. Composition selon la revendication 1, qui comprend en outre au moins un agent aidant à pénétrer la surface et/ou un ou plusieurs chélateurs.

3. Composition selon la revendication 1 ou la revendication 2, dans laquelle ledit agent photochimiothérapeutique est présent à raison d'une concentration de 0,01 à 50% en poids.

4. Composition selon l'une quelconque des revendications 1 à 3 dans laquelle ledit agent qui adhère à la muqueuse est présent à raison d'une concentration de 0,05 à 50% en poids.

5. Composition selon l'une quelconque des revendications 1 à 4, dans laquelle ledit agent est un composé de formule I :
R² ₂N-CH₂COCH₂-CH₂CO-OR¹ (I)
(dans laquelle
R¹ et R² représentent chacun indépendamment un atome d'hydrogène ou un groupe alkyle à chaîne droite, ramifiée ou cyclique, facultativement substitué, qui peut facultativement être interrompu par un ou plusieurs groupes -O-, -NR³-, -S- ou -PR³-, à condition que R¹ soit différent d'un atome d'hydrogène ;
et R³ représente un atome d'hydrogène ou un groupe alkyle en C₁₋₆
ou un sel pharmaceutiquement acceptable de ce composé.

6. Composition selon la revendication 5, dans laquelle dans la formule I, R¹ représente soit un groupe alkyle non substitué (par exemple un groupe alkyle en C₁₋₆) soit un groupe alkyle (par exemple un groupe alkyle en C₁₋₂) substitué par un groupe aryle (par exemple un groupe phényle) et/ou chaque R² représente un atome d'hydrogène.

7. Composition selon la revendication 5 ou la revendication 6, dans laquelle R¹ représente un groupe alkyle en C₁₋₁₆.

8. Composition selon la revendication 7, dans laquelle R¹ représente un hydrocarbure saturé à chaîne droite choisi parmi les groupes hexyle, heptyle et octyle.

9. Composition selon la revendication 5, dans laquelle dans la formule I, R¹ représente un groupe benzyle substitué et chaque R² représente un atome d'hydrogène.

10. Composition selon la revendication 9, dans laquelle ledit agent photochimiothérapeutique représente l'ester de p-isopropylbenzyl-ALA, l'ester de p-méthylbenzyl-ALA, l'ester de p-(tri-fluorométhyl)-benzyl-ALA, l'ester de p-(t-butyl)benzyl-ALA, l'ester de p-nitrobenzyl-ALA, l'ester de p-fluorobenzyl-ALA, l'ester de 2-fluorobenzyl-ALA, l'ester de 2,3,4,5,6-pentafluorobenzyl-ALA, l'ester de 4-chlorobenzyl-ALA, l'ester de 3-nitrobenzyl-ALA, l'ester de 3-fluorobenzyl-ALA, l'ester de 4-méthoxybenzyl-ALA, l'ester de 2-méthylbenzyl-ALA ou l'ester de 3-méthylbenzyl-ALA.

11. Composition selon l'une quelconque des revendications 1 à 4, dans laquelle l'ester d'ALA est l'ester méthylique de 5-ALA, l'ester hexylique de 5-ALA ou l'ester benzylique de 5-ALA.

12. Composition selon l'une quelconque des revendications précédentes, dans laquelle ledit agent qui adhère à la muqueuse est un polymère pouvant gonfler en présence d'eau et qui peut former des liaisons hydrogène.

13. Composition selon la revendication 12, dans laquelle ledit polymère est choisi parmi des polymères refermant un poly(acide carboxylique), des dérivés cellulosiques ou des esters ou des éthers de cellulose, des gommes, des argiles, des polysaccharides, des amidons, des formulations lipophiles renfermant des polysaccharides, des glucides facultativement substitués par des groupes sulfate, phosphate, sulfonate ou phosphonate, des polypeptides, de la chitosane (lactate ou glutamate) ou de la carboxyméthylchitine, des glycosaminoglycanes, des métaux ou des sels hydrosolubles de l'acide alginique, du schléroglucane, des agglutinants renfermant de l'oxyde de bismuth ou de l'oxyde d'aluminium, des athérocollagènes, des polymères polyvinyliques, des polysiloxanes, des polyéthers, des poly(oxydes d'éthylène) et des polyéthylèneglycols, des polyalcoxys et des polyacrylamides, et les dérivés et les sels de ces composés.

14. Composition selon la revendication 13, dans laquelle ledit agent qui adhère à la muqueuse est choisi parmi le poly(acide (acrylique, maléique, itaconique, citraconique, hydroxyéthylméthacrylique ou méthacrylique)), la méthylcellulose, l'éthylcellulose, la méthyléthylcellulose, l'hydroxyméthylcellulose, l'hydroxyéthylcellulose, l'hydroxypropylcellulose, l'hydroxyéthyléthylcellulose, la carboxyméthylcellulose, l'hydroxypropylméthylcellulose, la gomme de xanthane, la gomme de guar, la gomme de caroube, les gommes adragantes, la gomme karaya, la gomme ghatti, la gomme cholla, la gomme de psyllium, la gomme arabique, le Veegun, l'attapulgite, le dextrane, la pectine, l'amylopectine, le dextrose, la mannane, le poly(acide polygalacturonique), l'amidon hydroxypropylique, le carboxyméthylamidon, l'Orabase™, l'octasulfate de sucrose, la caséine, le gluten, la gélatine, la colle de fibrine, l'acide hyaluronique, l'alginate de sodium ou l'alginate de magnésium, des poly(alcools de vinyle), des polyvinylméthyléthers, la polyvinylpyrrolidone, des polymères vinyliques polycarboxylés, et les dérivés et les sels de ces composés.

15. Composition selon la revendication 1 conjointement avec un agent qui adhère à la muqueuse choisi parmi des hydrogels polyacryliques, la chitosane, le poly(alcool de vinyle), l'hydroxypropylcellulose, l'hydroxypropylméthylcellulose, l'alginate de sodium, le scléroglucane, la gomme de xanthane, la pectine, le poly(acide galacturonique) et l'Orabase™.

16. Composition selon l'une quelconque des revendications 1 à 15, dans laquelle ladite composition est sous la forme d'un onguent, d'un gel ou d'une crème avec une base aqueuse.

17. Composition telle que définie dans une quelconque revendication précédente, destinée à être utilisée en tant que médicament.

18. Composition selon la revendication 17, destinée à être utilisée dans le traitement ou le diagnostic des troubles ou des anomalies des surfaces épithéliales internes.

19. Composition selon la revendication 18, dans laquelle lesdites surfaces épithéliales internes sont des surfaces internes de la muqueuse.

20. Composition selon la revendication 19, dans laquelle lesdites surfaces internes de la muqueuse sont l'intérieur de la bouche, du pharynx, de l'oesophage, de l'estomac, des intestins et des appendices de l'intestin, du rectum, du canal anal, des voies nasales, des sinus nasaux, du nasopharynx, de la trachée, des bronches, des bronchioles, de l'uretère, de la vessie urinaire, de l'urètre, du vagin, du col de l'utérus ou de l'utérus.

21. Composition selon l'une quelconque des revendications 18 à 20, destinée à être utilisée dans le traitement des anomalies ou des troubles malins, pré-malins et bénins sensibles à la photochimiothérapie, de préférence des tumeurs ou autres croissances, en particulier les épithéliomas basocellulaires, la dysplasie ou autres croissances, et d'autres maladies ou infections, par exemple des infections bactériennes, virales ou fongiques, par exemple les infections liées au virus de l'herpès.

22. Utilisation d'un agent photochimiothérapeutique tel que défini dans l'une quelconque des revendications 1 ou 5 à 11, conjointement avec un agent qui adhère la muqueuse tel que défini dans l'une quelconque des revendications 1 ou 12 à 15, facultativement conjointement avec au moins un agent aidant à pénétrer la surface et facultativement avec un ou plusieurs chélateurs, dans la préparation d'une composition destinée au traitement ou au diagnostic de troubles ou d'anomalies tels que définis dans la revendication 18 ou 21 des surfaces épithéliales internes telles que définies dans l'une quelconque des revendications 18 à 20.

23. Produit comprenant un agent photochimiothérapeutique tel que défini dans l'une quelconque des revendications 1 ou 5 à 11, et un agent qui adhère la muqueuse tel que défini dans l'une quelconque des revendications 1 ou 12 à 15, facultativement conjointement avec au moins un agent aidant à pénétrer la surface et facultativement un ou plusieurs chélateurs, en tant que préparation combinée destinée à être utilisé de façon simultanée, séparée ou séquentielle dans le traitement ou le diagnostic de troubles ou d'anomalies tels que définis dans la revendication 18 ou 21 des surfaces épithéliales internes telles que définies dans l'une quelconque des revendications 18 à 20.

24. Utilisation d'un agent photochimiothérapeutique tel que défini dans l'une quelconque des revendications 1 ou 5 à 11 et d'un agent qui adhère la muqueuse tel que défini dans l'une quelconque des revendications 1 ou 12 à 15, facultativement conjointement avec au moins un agent aidant à pénétrer la surface et facultativement avec un ou plusieurs chélateurs, dans la préparation d'un produit destiné à être utilisé de façon simultanée, séparée ou séquentielle dans le traitement ou le diagnostic de troubles ou d'anomalies tels que définis dans la revendication 18 ou 21 des surfaces épithéliales internes telles que définies dans l'une quelconque des revendications 18 à 20.

25. Trousse destinée à étre utilisée dans la photochimiothérapie ou le diagnostic de troubles ou d'anomalies des surfaces épithéliales internes du corps, de préférence telles que définies dans la revendication 19 ou 20, comprenant :
a) un premier récipient contenant un agent photochimiothérapeutique, tel que défini dans l'une quelconque des revendications 1 ou 5 à 11, ou un sel pharmaceutiquement acceptable de cet agent,
b) un deuxième récipient contenant un agent qui adhère à la muqueuse, tel que défini dans l'une quelconque des revendications 1 ou 12 à 15, ou un sel pharmaceutiquement anceptable de cet agent,
c) un troisième récipient contenant au moins un agent aidant à pénétrer la surface ; et facultativement
d) un ou plusieurs chélateurs contenus dans l'un dudit premier, deuxième ou troisième récipient ou dans un quatrième récipient.

26. Procédé de diagnostic in vitro d'anomalies ou de troubles des surfaces épithéliales internes, de préférence telles que définis dans la revendication 19 ou 20, par l'analyse d'un échantillon de fluide biologique ou de tissu cellulaire d'un patient, ledit procédé comprenant au moins les étapes suivantes :
i) mélanger ledit fluide biologique ou ledit tissu cellulaire avec une composition telle que définie dans l'une quelconque des revendications 1 à 16,
ii) exposer ledit mélange à la lumière,
iii) vérifier le niveau de fluorescence, et
iv) comparer le niveau de la fluorescence aux niveaux témoins.
